# EUROPEAN PATENT SPECIFICATION

(11) **EP 3 950 056 B1**
(45) Date of publication and mention of the grant of the patent: **23.11.2022**
(21) Application number: 20782537.3
(22) Date of filing: 26.02.2020
(51) Int. Cl.: A61P 1/02, A61L 26/00, A61K 47/14, A61K 47/18, A61K 47/24, A61K 47/30, A61K 47/34

(54) **BIOLOGICAL MATERIAL**
BIOLOGISCHES MATERIAL
MATÉRIAU BIOLOGIQUE

(30) Priority: 29.03.2019 JP 2019065252
(43) Date of publication of application: 09.02.2022
(73) Proprietor: FUJIFILM Corporation, Tokyo 106-8620 (JP)
(72) Inventor: CHIBA Kosuke, Ashigarakami-gun, Kanagawa 258-8577 (JP); TAKAKU Koji, Ashigarakami-gun, Kanagawa 258-8577 (JP); TOMIKAWA Haruki, Ashigarakami-gun, Kanagawa 258-8577 (JP)
(74) Representative: HGF
(86) International application number: PCT/JP2020/007834
(87) International publication number: WO 2020/202926

(56) References cited:
- WO-A1-2018/073740
- CN-A- 102 579 341
- JP-A- 2003 517 886
- JP-A- 2005 529 949
- JP-A- 2008 528 463
- JP-A- 2010 536 838
- US-A1- 2014 186 279
- LAREDJ-BOUREZG F ET AL: "Pickering Emulsions Stabilised by Biodegradable Particles Offer a Double Level of Controlled Delivery of Hydrophobic Drugs", ISSUES IN TOXICOLOGY, ROYAL SOCIETY OF CHEMISTRY, CAMBRIDGE, PAGE(S) 143 - 156 , 6 December 2013 (2013-12-06), XP009529739, ISSN: 1757-7179 ISBN: 978-1-84973-398-4 Retrieved from the Internet: URL:https://ebookcentral.proquest.com/lib/ epo-ebooks/reader.action?docID=1713684&ppg =164
- F. Laredj-Bourezg , M.-A. Bolzinger , Jean Pelletier , M.-R. Rovere , B. Smatti , Y. Chevalier: "Pickering Emulsions Stabilised by Biodegradable Particles Offer a Double Level of Controlled Delivery of Hydrophobic Drugs", Issues in Toxicology, vol. 20, 2014, pages 143-156, XP009529739, ISSN: 1757-7179, DOI: 10.1039/9781849734639-00143 ISBN: 978-1-84973-398-4

## Description

### BACKGROUND OF THE INVENTION

### 1. Field of the Invention

The present invention relates to a biomaterial.

### 2. Description of the Related Art

In cancer patients, cancer treatment affects the mucous membrane of the mouth and, stomatitis occurs easily. For example, in anti-cancer drug treatment, in radiation therapy for head and neck cancer (cancer in the range from the head to the neck) in a case where a drug that easily causes stomatitis is administered, when radiation is directly applied to the mucous membrane of the mouth, stomatitis is inevitable. The pain of stomatitis is so strong that it is difficult to eat a meal by mouth.

As a symptomatic treatment for stomatitis, a patch (for example, Aphthaseal^{(R)} 25 µg, manufactured by Taisho Pharmaceutical Co., Ltd., active ingredient: triamcinolone acetonide) that is directly attached to the affected part, an ointment (for example, Dexaltin oral ointment for oral cavity, manufactured by Nippon Kayaku Co., Ltd., active ingredient: dexamethasone) that is applied to the affected part, and a spray agent (for example, Salcoat^{(R)} capsule for oral spray 50µg, manufactured by TEIJIN PHARMA LIMITED, active ingredient: beclomethasone propionate ester) that is sprayed on the affected part are mentioned.

However, these therapeutic agents contain steroids which are immunosuppressive agents, as active ingredients, and thus are not desirable for cancer patients.

In addition, when eating a meal by mouth, the patch attached to the affected part may be peeled off, or the ointment or the spray agent applied to the affected part may be lost, and thus the pain of stomatitis cannot be suppressed.

Accordingly, a material capable of suppressing such pain of stomatitis has been desired.

For example, JP2010-536838A discloses "a non-aqueous pharmaceutical pre-preparation containing a low viscosity mixture of i) a non-polymeric and controlledly releasable matrix, ii) at least one biocompatible (preferably containing oxygen) organic solvent, iii) at least one active peptide substance, and iv) at least one fat-soluble acid" (claim 1).

Further, JP2009-516724A discloses "an oil-in-water type emulsion which contains an active ingredient present in a range between 0.00001% and 79% with respect to the entire composition, in which oil droplets having a diameter in a range of 5 nm to several hundred microns exhibit a self-assembled nanosized structure of a hydrophilic domain having a size of a diameter in a range of 0.5 to 200 nm due to the presence of a lipophilic additive" (claim 1). US 2014/186279 A1 discloses topical bioadhesive formulations comprising low viscosity, non-liquid crystalline, mixtures of: a) at least one neutral diacyl lipid and/or at least one tocopherol; b) at least one phospholipid; c) at least one biocompatible, oxygen containing, low viscosity organic solvent; wherein at least one bioactive agent is dissolved or dispersed in the low viscosity mixture and wherein the pre-formulation forms, or is capable of forming, at least one liquid crystalline phase structure upon contact with an aqueous fluid.

### SUMMARY OF THE INVENTION

However, in the non-aqueous pharmaceutical pre-preparation disclosed in JP2010-536838A and the oil-in-water type emulsion disclosed in JP2009-516724A, the retention (the attachability to the mucous membrane in a moist environment) in a case of being brought into contact with water was not at a satisfactory level as shown by Comparative Examples described later, and there was room for improvement in these compositions.

An object of the present invention is to provide a biomaterial that is excellent in retention in a case of being brought into contact with water.

As a result of diligent studies to solve the above problems, the inventors of the present invention have found that the above problems can be solved by using a biomaterial containing predetermined components and have completed the present invention.
(1) A biomaterial comprising:
   a neutral acyl lipid;
   a phospholipid; and
   an amphipathic block copolymer which contains a hydrophilic segment and a hydrophobic segment and in which a difference in ClogP value between the hydrophilic segment and the hydrophobic segment is more than 1.00,
   in which a content of the amphipathic block copolymer is 1.0% to 20% by mass with respect to a total mass of a solid content of the biomaterial.
(2) The biomaterial according to (1), in which the amphipathic block copolymer has a number average molecular weight of 1,000 to 10,000, preferably a number average molecular weight of 1,000 to 5,000.
(3) The biomaterial according to (1) or (2), in which the neutral acyl lipid contains 30% to 100% by mass of a neutral monoacyl lipid, 0% to 70% by mass of a neutral diacyl lipid, and 0% to 20% by mass of a neutral triacyl lipid, with respect to a total mass of the neutral acyl lipid.
(4) The biomaterial according to any one of (1) to (3), in which an acyl group of the neutral acyl lipid has 6 to 32 carbon atoms; preferably 16 to 22 carbon atoms.
(5) The biomaterial according to any one of (1) to (4), further comprising a quaternary ammonium salt.
(6) The biomaterial according to (5) wherein a content of the phospholipid and quaternary ammonium salt is 25% to 45% by mass with respect to the total mass of the solid content of the biomaterial.
(7) The biomaterial according to any one of (1) to (6), in which an acyl group of the phospholipid has 12 to 22 carbon atoms; preferably 16 to 18 carbon atoms.
(8) The biomaterial according to (5), in which a quaternary ammonium cation of the quaternary ammonium salt is represented by a formula described later.
(9) The biomaterial according to (8), in which the alkoxy group, the acyl group, or the acyloxy group, with which the hydrogen atom of the alkyl group is substituted, has 16 to 18 carbon atoms.
(10) The biomaterial according to any one of (1) to (9), in which the neutral acyl lipid is an acyl glycerol.
(11) The biomaterial according to (10), in which the acyl glycerol is a glyceryl oleate.
(12) The biomaterial according to any one of (1) to (11), in which the difference in ClogP value between the hydrophilic segment and the hydrophobic segment is more than 2.00.
(13) The biomaterial according to any one of (1) to (12), in which the hydrophobic segment contains polycaprolactone.
(14) The biomaterial according to any one of (1) to (13), in which the amphipathic block copolymer is a triblock copolymer.
(15) The biomaterial according to (14), in which the triblock copolymer has a constitution of polycaprolactone-polytetrahydrofuran-polycaprolactone.
(16) The biomaterial according to any one of (1) to (15), wherein a content of water is 1.0% by mass or less with respect to a total mass of the biomaterial.
(17) The biomaterial according to any one of (1) to (16), in which the biomaterial forms a liquid crystal phase in a case of being brought into contact with water.
(18) The biomaterial according to (17), in which the liquid crystal phase is any one selected from the group consisting of a hexagonal columnar phase, a lamellar phase, a sponge phase, a bicontinuous cubic phase, and a mixed state of two or more thereof.
(19) The biomaterial according to any one of (1) to (18), in which the biomaterial is used for mucous membrane protection.
(20) The biomaterial according to (19), in which the biomaterial is used for oral cavity mucous membrane protection.

Also described is the biomaterial according to any one of (1) to (20), in which the biomaterial is used for a sustained release of a drug.

According to the present invention, it is possible to provide a biomaterial that is excellent in retention in a case of being brought into contact with water.

### DESCRIPTION OF THE PREFERRED EMBODIMENTS

In the present specification, the range indicated by using "to" means a range including both ends before and after "to".

For example, a range indicated by "A to B" includes A and B.

In addition, in the present specification, the solid content is intended to be a component contained in the biomaterial excluding the dispersion medium component, and a component is calculated as a solid content even in a case where the property thereof is liquid.

A biomaterial according to the present invention contains a neutral acyl lipid and a predetermined amphipathic block copolymer.

The biomaterial according to the invention is excellent in retention in a case of being brought into contact with water. Further, in the biomaterial according to the invention, since the amphipathic block copolymer is used, the viscosity is improved, the occurrence of "dripping" is suppressed, and the usability is improved.

Hereinafter, each component of the biomaterial according to the present invention will be described.

### <Neutral acyl lipid>

The neutral acyl lipid means an electrically neutral acyl lipid. That is, the neutral acyl lipid does not include a cation moiety and an anion moiety. The acyl lipid means a lipid containing an acyl group.

Examples of the neutral acyl lipid include a neutral monoacyl lipid, a neutral diacyl lipid, and a neutral triacyl lipid.

In the biomaterial according to the present invention, the neutral acyl lipid contains a neutral monoacyl lipid and a neutral diacyl lipid, and the total content of the neutral monoacyl lipid and the neutral diacyl lipid is preferably 80% to 100% by mass, more preferably 90% to 100% by mass, and still more preferably 100% by mass, with respect to the total mass of the neutral acyl lipid.

The content of the neutral monoacyl lipid is not particularly limited; however, it is preferably 30% to 100% by mass, more preferably 40% to 100% by mass, and still more preferably 40% to 80% by mass, with respect to the total mass of the neutral acyl lipid.

The content of the neutral diacyl lipid is not particularly limited; however, it is preferably 0% to 70% by mass, more preferably 0% to 60% by mass, and still more preferably 20% to 60% by mass, with respect to the total mass of the neutral acyl lipid.

The content of the neutral triacyl lipid is not particularly limited; however, it is preferably 0% to 20% by mass and more preferably 0% to 10% by mass with respect to the total mass of the neutral acyl lipid.

Among the above, with respect to the total mass of the neutral acyl lipid, it is preferable that 40% to 100% by mass of the neutral monoacyl lipid is contained, 0% to 60% by mass of the neutral diacyl lipid is contained, and 0% to 20% by mass of the neutral triacyl lipid is contained, and it is more preferable that 40% to 80% by mass of the neutral monoacyl lipid is contained, 20% to 60% by mass of the neutral diacyl lipid is contained, and 0% to 10% by mass of the neutral triacyl lipid is contained, since the liquid crystal phase to be formed exhibits a more preferred phase (for example, a hexagonal columnar (H2) phase).

The number of carbon atoms of the acyl group of the neutral acyl lipid is not particularly limited; however, the acyl group preferably has 6 to 32 carbon atoms and more preferably 16 to 22 carbon atoms.

The hydrocarbon group as the acyl group, excluding a carbonyl group, is preferably a saturated or unsaturated chain-type hydrocarbon group having 16 to 32 carbon atoms, and more preferably a saturated or unsaturated chain-type hydrocarbon group having 6 to 22 carbon atoms. Examples of the hydrocarbon group include CH₃(CH₂)₁₄-, CH₃(CH₂)₇CH=CH(CH₂)₇-, and CH₃(CH₂)₄(CH=CHCH₂)₂(CH₂)₆-.

In a case where a neutral acyl lipid has two or more acyl groups in one molecule, the kinds of acyl groups may be the same or different from each other.

The neutral acyl lipid is a lipid obtained by forming an ester bond between a polyol such as glycerol, diglycerol, sugar (for example, inositol), or succinic acid, and a fatty acid. Among the above, an acyl glycerol is preferable and a glyceryl oleate is more preferable.

Examples of the acyl glycerol include monoacyl glycerol, diacyl glycerol, and triacyl glycerol.

In addition, examples of the glyceryl oleate include glyceryl monooleate, glyceryl dioleate, and glyceryl trioleate.

Here, the monoacyl glycerol and the glyceryl monooleate correspond to one aspect of the neutral monoacyl lipid described above, and the diacyl glycerol and the glyceryl dioleate correspond to one aspect of the neutral diacyl lipid described above, and the triacyl glycerol and the glyceryl trioleate correspond to one aspect of the neutral triacyl lipid.

In the present specification, the content proportions of the neutral monoacyl lipid, the neutral diacyl lipid, and the neutral triacyl lipid in the neutral acyl lipid are obtained from measurement with a high performance liquid chromatography (HPLC) method.
• HPLC measurement conditions
Column: Imtakt Cadenza CD-C18 (4.6 mm x 300 mm)
Eluent: water/tetrahydrofuran
Flow rate: 1.0 mL/min
Detection: CAD (corona charged particles)
Column temperature: 50°C

The content of the neutral acyl lipid in the biomaterial according to the present invention is not particularly limited; however, it is preferably 35% to 85% by mass and more preferably 55% to 75% by mass, with respect to the total mass of the solid content of the biomaterial according to the present invention.

### <Amphipathic block copolymer>

The biomaterial according to the invention contains an amphipathic block copolymer.

The amphipathic block copolymer is a block copolymer containing a hydrophilic segment and a hydrophobic segment. For this reason, in a case where an amphipathic block copolymer is used for the biomaterial according to the invention, the hydrophilic segment is arranged in the core (the water phase) of the liquid crystal micelle formed from the neutral acyl lipid, and the hydrophobic segment is arranged in the oil phase. In a case where the hydrophilic segment and the hydrophobic segment of the amphipathic block copolymer are arranged in the water phase and the oil phase of the liquid crystal micelle in this manner, the amphipathic block copolymer acts as a linker of the liquid crystal micelle to stabilize the liquid crystal micelle, whereby the liquid crystal phase is highly ordered, and the retention of the biomaterial according to the invention is improved in a case where the biomaterial is brought into contact with water. Furthermore, in a case where the amphipathic block copolymer is used, the viscosity of the material in the state before being brought into contact with water (the pharmaceutical pre-preparation state) is increased, and thus the dripping thereof can be suppressed.

The segment is a structural unit that constitutes each block in the block copolymer and is composed of a specific repeating unit.

The hydrophilic segment is a segment that exhibits hydrophilicity. Specifically, the hydrophilic segment means a segment having a ClogP value of less than -0.60. From the viewpoint that the effects of the present invention are more excellent, the ClogP value of the hydrophilic segment is preferably -0.70 or less and more preferably -0.80 or less. The lower limit thereof is not particularly limited; however, it is preferably -3.00 or more.

The hydrophobic segment is a segment that exhibits hydrophobicity. Specifically, the hydrophobic segment means a segment having a ClogP value of -0.60 or more. From the viewpoint that the effects of the present invention are more excellent, the ClogP value of the hydrophobic segment is preferably 0.00 or more and more preferably 1.00 or more. The upper limit thereof is not particularly limited; however, it is preferably 4.00 or less.

The above definitions use polypropylene oxide (PPO) as the hydrophobic segment (Journal of Japan Oil Chemists' Society, Vol. 49, No. 10 (2010)) and are made based on the fact that the ClogP value of PPO is -0.55.

The ClogP value is a partition coefficient, which is based on the ratio between concentrations of an organic compound partitioned to water and octanol in the two phase system of water and octanol, and is an indicator indicating the hydrophobicity of the organic compound.

In the present specification, the ClogP value of the segment is a value obtained by calculating the ClogP value of the trimer of the repeating unit constituting the segment by using the ChemOffice Professional 2016.

The trimer of the repeating unit constituting the segment corresponds to a compound which is obtained by connecting three repeating units and arranging hydrogen atoms at both terminals. More specifically, for example, the ClogP value of a hydrophilic segment consisting of polyethylene oxide of which the repeating unit is represented by-(OCH₂CH₂)-corresponds to a ClogP value of a compound represented by H-(OCH₂CH₂)-(OCH₂CH₂)-(OCH₂CH₂)-H, which is obtained by connecting three repeating units and arranging hydrogen atoms at both terminals.

The amphipathic block copolymer is not limited as long as it has a hydrophilic segment and a hydrophobic segment. For example, it may be a diblock copolymer consisting of one hydrophilic segment and one hydrophobic segment (so-called an AB type block copolymer) or may be a triblock copolymer (so-called an ABA type block copolymer) consisting of three segments such as hydrophilic segment - hydrophobic segment - hydrophilic segment or hydrophobic segment - hydrophilic segment - hydrophobic segment.

The kind of the polymer constituting the hydrophilic segment is not particularly limited; however, examples thereof include polyethylene oxide (PEO) (ClogP value: -1.48) and polytetrahydrofuran (PTH) (ClogP value: -0.81).

The kind of the polymer constituting the hydrophobic segment is not particularly limited; however, examples thereof include polylactic acid (PLA) (ClogP value: 2.46), polycaprolactone (PCL) (ClogP value: 0.19), and polypropylene oxide (PPO) (ClogP value: -0.55).

The difference in ClogP value between the hydrophilic segment and the hydrophobic segment is more than 1.00. Among the above, the difference in ClogP value is preferably more than 2.00 and more preferably 3.00 or more from the viewpoint that the effects of the present invention are more excellent. The upper limit thereof is not particularly limited; however, it is preferably 5.00 or less and more preferably 4.50 or less.

Examples of the amphipathic block copolymers include a polyethylene oxide (PEO) - polylactic acid (PLA) diblock copolymer, a polycaprolactone (PCL) - polyethylene oxide (PEO) diblock copolymer, a polycaprolactone (PCL) - polyethylene oxide (PEO) - polycaprolactone (PCL) triblock copolymer, and a polycaprolactone (PCL) - polytetrahydrofuran (PTHF) - polycaprolactone (PCL) triblock copolymer.

The number average molecular weight of the amphipathic block copolymer is not particularly limited, however, it is preferably 1,000 to 10,000 and more preferably 1,000 to 5,000 from the viewpoint that the effects of the present invention are more excellent.

In the present invention, the number average molecular weight of the amphipathic block copolymer is measured by gel permeation chromatography (GPC) under the following measurement conditions.
Column: TOSOH TSK guardcolumn Super HZ-H
TOSOH TSK gel Super HZM-H
TOSOH TSK gel Super HZ 4000
TOSOH TSK gel Super HZ 2000
Eluent: tetrahydrofuran (containing a stabilizer)
Flow rate: 0.35 mL/min
Temperature: 40°C
Detector: refractive index detector (RI)

The content of the amphipathic block copolymer in the biomaterial according to the invention is 1.0% to 20% by mass and is preferably 5.0% to 15% by mass with respect to the total mass of the solid content of the biomaterial according to the invention.

### <Other components>

The biomaterial according to the embodiment of the present invention may contain components other than those described above.

### <Phospholipid>

The biomaterial according to the invention contains a phospholipid.

The phospholipid is not particularly limited as long as it has a phosphate ester structure in the molecular structure thereof; however, a glycerophospholipid having glycerin as a skeleton and a sphingophospholipid having sphingosine as a skeleton are typical.

In both a case of the phospholipid being a glycerophosphoric acid and a case of the phospholipid being a sphingophospholipid, an acyl group derived from a fatty acid is contained in the molecule.

The number of carbon atoms of the acyl group of the phospholipid is not particularly limited; however, the acyl group preferably has 12 to 22 carbon atoms and more preferably 16 to 18 carbon atoms.

The hydrocarbon group as the acyl group, excluding a carbonyl group, is preferably a saturated or unsaturated chain-type hydrocarbon group having 11 to 21 carbon atoms, and more preferably a saturated or unsaturated chain-type hydrocarbon group having 15 to 17 carbon atoms. Examples of the hydrocarbon group include CH₃(CH₂)₁₄-, CH₃(CH₂)₇CH=CH(CH₂)₇-, and CH₃(CH₂)₄(CH=CHCH₂)₂(CH₂)₆-.

In a case where a phospholipid has two or more acyl groups in one molecule, the kinds of acyl groups may be the same or different from each other.

Specific examples of the phospholipid include phosphatidylcholine. The acyl group of the phosphatidylcholine is preferably an acyl group derived from palmitic acid (CH₃(CH₂)₁₄COOH), oleic acid (CH₃(CH₂)₇CH=CH(CH₂)₇COOH), or linoleic acid (CH₃(CH₂)₄(CH=CHCH₂)₂(CH₂)₆COOH). Specific examples of the phosphatidylcholine include a PO phosphatidylcholine (a phosphatidylcholine having palmitic acid at the first position (the α position), oleic acid at the second position (the β position), and choline at the third position (the γ position)), a DL phosphatidylcholine (a phosphatidylcholine having linoleic acid at the first position (the α position), linoleic acid at the second position (the β position), and choline at the third position (the γ position)), and a dipalmitoyl phosphatidylcholine.

The content of the phospholipid in the biomaterial according to the present invention is not particularly limited; however, the content thereof in total together with a quaternary ammonium salt described later is preferably 15% to 65% by mass and more preferably 25% to 45% by mass with respect to the total mass of the solid content of the biomaterial according to the present invention.

### (Quaternary ammonium salt)

The biomaterial according to the embodiment of the invention may contain a quaternary ammonium salt.

The quaternary ammonium salt is an ionic compound consisting of a quaternary ammonium cation and an anion.

The quaternary ammonium cation is a polyatomic ion having a positive charge represented by the molecular formula NR₄⁺. Here, R's each independently represent an alkyl group or an aryl group, and a plurality of R's may be the same or different from each other.

Among the above, the quaternary ammonium cation of the quaternary ammonium salt is preferably a quaternary ammonium cation represented by the following formula.

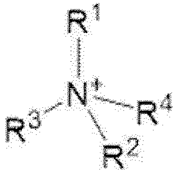

In the formula, R¹, R², R³, and R⁴ are each independently an alkyl group having 1 to 22 carbon atoms, and a hydrogen atom of the alkyl group may be substituted with an alkoxy group, an acyl group, or an acyloxy group.

The alkoxy group, the acyl group, or the acyloxy group, with which the hydrogen atom of the alkyl group is substituted, preferably has 16 to 18 carbon atoms.

The quaternary ammonium salt is preferably a di-long-chain alkyl quaternary ammonium salt.

The di-long-chain alkyl quaternary ammonium salt is a salt in which two of R's of a quaternary ammonium cation represented by the molecular formula NR₄⁺ are a long-chain alkyl group. The other two R's are independently a short-chain alkyl or an aryl group. Here, the long-chain alkyl group represents an alkyl group having 8 or more carbon atoms, and the short-chain alkyl group represents an alkyl group having 1 to 7 carbon atoms.

In the biomaterial according to the embodiment of the invention, the long-chain alkyl group of the quaternary ammonium cation of the di-long-chain alkyl quaternary ammonium salt preferably has 12 to 22 carbon atoms and more preferably 16 to 18 carbon atoms.

Examples of such a long-chain alkyl group include a hexadecyl group, a heptadecyl group, and an octadecyl group. The kinds of the two long-chain alkyl groups may be the same or different from each other.

Examples of the quaternary ammonium salt include dioleoyloxytrimethylammonium propane chloride (DOTAP), dioctadecenyltrimethylammonium propane chloride (DOTMA), didecylmethylammonium chloride, didecyldimethylammonium bromide, dicocoyldimethylammonium chloride, dicocoyldimethylammonium bromide, dilauryldimethylammonium chloride, disetyldimethylammonium chloride, disetyldimethylammonium bromide, distearyldimethylammonium chloride, distearyldimethylammonium bromide, dioleyldimethylammonium chloride, dibehenyldimethylammonium chloride, dibehenyldimethylammonium bromide, dialkyldimethylammonium chloride, dialkyldimethylammonium bromide, dicocoylethylhydroxyethylmonium metosulfate, dipalmitoylethylhydroxyethylmonium metosulfate, and distearoylethylhydroxyethylmonium metosulfate.

In addition, these quaternary ammonium salt can be used as a mixture of two or more kinds thereof.

The content of the quaternary ammonium salt in the biomaterial according to the embodiment of the present invention is not particularly limited; however, the content thereof in total together with phospholipid described above is preferably 15% to 65% by mass and more preferably 25% to 45% by mass with respect to the total mass of the solid content of the biomaterial according to the embodiment of the present invention.

### (Non-aqueous dispersion medium)

The biomaterial according to the embodiment of the invention may contain a non-aqueous dispersion medium.

The non-aqueous dispersion medium is not particularly limited, however, it is preferably an alcohol-based solvent and more preferably ethanol, cetyl alcohol, stearyl alcohol, or propylene glycol, since the adverse effect on the human body is not large.

The solid content concentration in the biomaterial according to the embodiment of the invention is not particularly limited; however, it is preferably 80% by mass or more and more preferably 90% by mass or more with respect to the total mass of the biomaterial according to the embodiment of the invention. The upper limit thereof is 100% by mass.

The biomaterial according to the embodiment of the present invention is preferably substantially free of water. The "substantially free of water" means that the water content is 1.0% by mass or less with respect to the total mass of the biomaterial. The water content is preferably 0.1% by mass or less with respect to the total mass of the biomaterial. The lower limit thereof is not particularly limited; however, it is 0% by mass.

The biomaterial according to the embodiment of the present invention can form a liquid crystal phase in a case of being brought into contact with water.

The water is not limited to pure water, and it may be water (aqueous moisture) contained in an aqueous fluid other than water. Examples of the aqueous fluid other than water include saliva, tissue fluid, blood, and lymph.

The amount of water which is brought into contact with the biomaterial according to the embodiment of the present invention is not particularly limited; however, it is preferably 20% to 1,000% by mass and more preferably 30% to 500% by mass, with respect to the total mass of the biomaterial according to the embodiment of the present invention.

Here, the liquid crystal phase is not particularly limited; however, it is usually any one phase selected from the group consisting of a hexagonal columnar (H2) phase, a lamellar (La) phase, a sponge (V2) phase, a bicontinuous cubic (L3) phase, and a mixed state of two or more thereof. The liquid crystal phase is preferably a hexagonal columnar phase.

The temperature at which the biomaterial according to the embodiment of the present invention is brought into contact with water is not particularly limited; however, it is preferably 20°C to 40°C and more preferably 35°C to 40°C.

### <Method of producing biomaterial>

The biomaterial according to the invention can be produced by mixing a neutral acyl lipid, an amphipathic block copolymer, and other components as desired.

The mixing method is not particularly limited, and a conventionally known mixing method can be used.

### <Use application and method of using biomaterial>

The biomaterial according to the embodiment of the present invention means a material that is used in a living body, and examples thereof include a material that is used in a living body, for example, for assisting or repairing a living body an original function of which has been lost or a living body a function of which has been deteriorated due to injury or disease.

The biomaterial according to the present invention can be used for mucous membrane protection, particularly preferably for oral cavity mucous membrane protection.

In a case where the biomaterial according to the present invention is used for a mucous membrane, when the biomaterial according to the present invention is placed on the mucous membrane and as necessary, water or a solution containing water is added thereon, the biomaterial according to the present invention absorbs water to form a liquid crystal phase, thereby strongly being attached to the mucous membrane.

In particular, in a case where the biomaterial according to the present invention is applied to the oral cavity mucous membrane, when the biomaterial according to the present invention is attached to the oral cavity mucous membrane, the liquid crystal phase is formed by the aqueous moisture in saliva, and thus is handling is easy. In addition, in a case where the amount of saliva is small, water may be sprayed to supply aqueous moisture after the biomaterial according to the present invention is attached to the oral cavity mucous membrane.

Since the biomaterial according to the invention has a relatively low viscosity, it can be attached to the mucous membrane by spray ejection.

### Examples

Hereinafter, the present invention will be more specifically described according to Examples, but the present invention is not limited to Examples below.

### [Examples 1 to 8 and Comparative Examples 1 to 9]

### (Preparation of biomaterial)

Each of the components shown in Table 1 was mixed according to the blending amount shown in Table 1 to prepare biomaterials of Examples 1 to 8 and Comparative Examples 1 to 8.

In Comparative Example 7, the pharmaceutical preparation A of Table 1 of JP2010-536838A was prepared and used as a biomaterial.

In Comparative Example 8, the pharmaceutical preparation B of Table 1 of JP2010-536838A was prepared and used as a biomaterial.

Further, in Comparative Example 9, a biomaterial was prepared according to the procedure of Example 1 of JP2009-516724A.

### <Result and evaluation>

### (Checking of formation of liquid crystal phase)

The prepared biomaterial (20 µL) was placed on a slide glass, water (about 200 µL) was sprayed thereon from above, excess water was removed therefrom, covered with a cover glass, and then an initial observation was carried out using a polarizing microscope (Nikon ECLIPSE E600 POL).

Thereafter, the biomaterial was heated together with the slide glass on a hot stage (INSTEC STC200) heated in advance to 40°C, and the change in the liquid crystal phase was checked.

### (Preparation of pseudo biological membrane)

Tetradodecyl ammonium bromide (TDAB, manufactured by FUJIFILM Wako Pure Chemical Corporation; 50 mg), polyvinyl chloride (manufactured by FUJIFILM Wako Pure Chemical Corporation; 800 mg), and di-n-octylphosphonate (DOPP, manufactured by FUJIFILM Wako Pure Chemical Corporation; 0.6 mL) were dissolved in tetrahydrofuran (THF, manufactured by FUJIFILM Wako Pure Chemical Corporation; 10 mL) to obtain a solution, which was then dried at room temperature with a petri dish to obtain a lipid membrane (about 200 µm thickness).

Next, the prepared lipid membrane was attached to a hydrogel consisting of agar (Karikorikan (registered trade mark), manufactured by Ina Food Industry Co., Ltd.; 0.5 g), gellan gum (Kelcogel (registered trade mark), manufactured by CP Kelco; 0.1 g), and distilled water (49.4 g).

Subsequently, the surface of the lipid membrane was coated with a 2-methacryloyloxyethyl phosphorylcholine (MPC) polymer (LIPIDURE (registered trade mark) - CM5206, manufactured by NOF CORPORATION) to obtain a pseudo biological membrane.

### (Evaluation of retention)

The prepared biomaterial was applied onto the prepared pseudo biological membrane (1 cmcp, 500 µm thickness), artificial saliva (Saliveht (registered trade mark), manufactured by TEIJIN PHARMA LIMITED) was sprayed thereon, and the biomaterial was allowed to stand for 1 minute such that water was absorbed, thereby preparing a sample for evaluation.

The prepared sample for evaluation was placed in a petri dish and filled with artificial saliva (Saliveht) until the sample for evaluation was immersed. The petri dish was placed in a constant temperature shaker (37°C) and shaken such that the sample for evaluation was repeatedly hit to the wall of the petri dish. In this test, the time required for the biomaterial, which absorbed water from the pseudo biological membrane, to disappear by being peeled or dissolved from the pseudo biological membrane was measured, and the retention was evaluated according to the following criteria. The evaluation results are shown in the column of "Evaluation" in Table 1.
AA: The material still remained after shaking for 6 hours.
A: The material disappeared after shaking for 6 hours.
B: The material disappeared after shaking for less than 6 hours.

### (Evaluation of viscosity)

The biomaterial was placed on a plate, and the viscosity was measured with a CP25-2 cone plate at a gap of 0.105 mm and a shear rate of 1 (1/s) using a rheometer (MCR302, manufactured by Anton Paar GmbH).

The viscosity of a liquid crystal material (Comparative Example 1) at a shear rate of 1 (1/s) was set to 1, where the liquid crystal material did not contain the amphipathic polymer, and the relative viscosity of each of Examples and Comparative Examples was evaluated according to the following criteria. The evaluation results are shown in the column of "Evaluation" in Table 1.
AA: more than two times
A: 1.5 times or more and less than 2 times
B: 1.1 times or more and less than 1.5 times
C: less than 1.1 times

In Table 1, each of the components is as follows.
Glyceryl oleate (1): 1-oleoyl-rac-glycerin (> 99%, manufactured by Sigma-Aldrich Co. LLC)
Glyceryl oleate (2) and glyceryl oleate(3): mixtures of glyceryl oleate (1) and (4)
Glyceryl oleate (4): Diolein (> 99%, manufactured by NU-CHECK-PREP Inc.)
Glyceryl oleate (5): Monoolein (> 40%, manufactured by Tokyo Chemical Industry Co., Ltd.)

### Phosphatidylcholine: Lipoid S100 (manufactured by Lipoid GmbH)

PLA-PEO-PLA: a triblock copolymer (manufactured by Sigma-Aldrich Co., LLC) consisting of three segments of polylactic acid - polyethylene oxide - polylactic acid
PCL-PEO: (manufactured by PolyScitech, a division of Akina, Inc.)
PCL-PEO-PCL: a triblock copolymer (manufactured by Nu Chemie, LLC) consisting of three segments of polycaprolactone-polyethylene oxide-polycaprolactone
PCL-PTTH-PCL: a triblock copolymer (manufactured by Sigma-Aldrich Co., LLC) consisting of three segments of polycaprolactone-polytetrahydrofuran-polycaprolactone
PEO-PPO-PEO (1): Pluronic P123 (manufactured by Sigma-Aldrich Co., LLC)
PEO-PPO-PEO (2): Pluronic L122 (manufactured by BASF SE)
PPO-PEO-PPO: Pluronic RPE1740 (manufactured by BASF SE)
PEG: polyethylene glycol (manufactured by FUJIFILM Wako Pure Chemical Corporation)
PE: polyethylene (manufactured by Sigma-Aldrich Co., LLC)

In Table 1, the kinds of liquid crystal phases are represented by the following abbreviations.
La: lamellar phase
H2: hexagonal columnar phase

The numerical value in the column of each of the components in Table 1 means a value in terms of "part by mass". For example, in Example 1, "10 parts by mass" of PLA-PEO-PLA was used.

In Table 1, "^{∗}1" in the column of "Comparative Example 7" means that the pharmaceutical preparation A of Table 1 of JP2010-536838A was used as a biomaterial.

In Table 1, "^{∗}2" in the column of "Comparative Example 8" means that the pharmaceutical preparation B of Table 1 of JP2010-536838A was used as a biomaterial.

Further, "^{∗}3" in the column of "Comparative Example 9" means a biomaterial prepared according to the procedure of Example 1 of JP2009-516724A.

**[Table 1]**

| | | Mono/di/Tri | Difference in ClogP value | Number average molecular weight | Example | | | | | | | |
|---|---|---|---|---|---|---|---|---|---|---|---|---|
| | | [% by mass] | | | 1 | 2 | 3 | 4 | 5 | 6 | 7 | 8 |
| Biomaterial | Glyceryl oleate (1) | 100/0/0 | - | - | - | - | - | - | - | 52 | - | - |
| | Glyceryl oleate (2) | 70/30/0 | - | - | - | - | - | - | - | - | 52 | - |
| | Glyceryl oleate (3) | 30/70/0 | - | - | - | - | - | - | - | - | - | 52 |
| | Glyceryl oleate (4) | 0/100/0 | - | - | - | - | - | - | - | - | - | - |
| | Glyceryl oleate (5) | 49/42/9 | - | - | 52 | 52 | 52 | 52 | 52 | - | - | - |
| | Phosphatidylcholine | - | - | - | 28 | 28 | 28 | 28 | 28 | 28 | 28 | 28 |
| | PLA-PEO-PLA | - | 1.67 | 2000 | 10 | - | - | - | - | - | - | - |
| | PCL-PEO | - | 3.96 | 4500 | - | 10 | - | - | - | - | - | - |
| | PCL-PEO-PCL | - | 3.96 | 3100 | - | - | 10 | - | - | | - | - |
| | PCL-PTHF-PCL | - | 3.27 | 3400 | - | - | - | 10 | 10 | 10 | 10 | 10 |
| | PEO-PPO-PEO (1) | - | 0.93 | 6100 | - | - | - | - | - | - | - | - |
| | PEO-PPO-PEO (2) | - | 0.93 | 2300 | - | - | - | - | - | - | - | - |
| | PPO-PEO-PPO | - | 0.93 | 3900 | - | - | - | - | - | - | - | - |
| | PEG | - | - | 2800 | - | - | - | - | - | - | - | - |
| | PE | - | - | 1700 | - | - | - | - | - | - | - | - |
| | Cetyl alcohol (dispersion medium) | - | - | - | 10 | 10 | 10 | 10 | - | 10 | 10 | 10 |
| | Ethanol (dispersion medium) | - | - | - | - | - | - | - | 10 | - | - | - |
| Result | Liquid crystal phase | | | | H2 | H2 | H2 | H2 | H2 | La | H2 | H2 |
| Evaluation | Viscosity | | | | A | A | A | A | A | AA | A | B |
| | Retention | | | | A | AA | AA | AA | AA | AA | AA | A |

**[Table 2]**

| | | Mono/di/Tri | Difference in ClogP value | Number average molecular weight | Com | | | parative Exam | | | ple | | |
|---|---|---|---|---|---|---|---|---|---|---|---|---|---|
| | | [% by mass] | | | 1 | 2 | 3 | 4 | 5 | 6 | 7 | 8 | 9 |
| Biomaterial | Glyceryl oleate (1) | 100/0/0 | - | - | - | - | - | - | - | - | ^{∗}1 | ^{∗}2 | ^{∗}3 |
| | Glyceryl oleate (2) | 70/30/0 | - | - | - | - | - | - | - | - | | | |
| | Glyceryl oleate (3) | 30/70/0 | - | - | - | - | - | - | - | - | | | |
| | Glyceryl oleate (4) | 0/100/0 | - | - | - | - | - | - | - | - | | | |
| | Glyceryl oleate (5) | 49/42/9 | - | - | 52 | 52 | 52 | 52 | 52 | 52 | | | |
| | Phosphatidylcholine | - | - | - | 28 | 28 | 28 | 28 | 28 | 28 | | | |
| | PLA-PEO-PLA | - | 1.67 | 2000 | - | - | - | - | - | - | | | |
| | PCL-PEO | - | 3.96 | 4500 | - | - | - | - | - | - | | | |
| | PCL-PEO-PCL | - | 3.96 | 3100 | - | - | - | - | - | - | | | |
| | PCL-PTHF-PCL | - | 3.27 | 3400 | - | - | - | - | - | - | | | |
| | PEO-PPO-PEO (1) | - | 0.93 | 6100 | - | 10 | - | - | - | - | | | |
| | PEO-PPO-PEO (2) | - | 0,93 | 2300 | - | - | 10 | - | - | - | | | |
| | PPO-PEO-PPO | - | 0,93 | 3900 | - | - | - | 10 | - | - | | | |
| | PEG | - | - | 2800 | - | - | - | - | 10 | - | | | |
| | PE | - | - | 1700 | - | - | - | - | - | 10 | | | |
| | Cetyl alcohol (dispersion medium) | | - | | 20 | 10 | 10 | 10 | 10 | 10 | | | |
| | Ethanol (dispersion medium) | - | - | - | - | - | - | - | - | - | | | |
| Result | Liquid crystal phase | | | | H2 | H2 | H2 | H2 | H2 | H2 | - | - | - |
| Evaluation | Viscosity | | | | C | B | C | C | C | C | - | - | - |
| | Retention | | | | B | B | B | B | B | B | B | B | B |

The biomaterials of Examples 1 to 8 had good retention.

Among the above, from the comparison of Examples 4 to 8, in a case where with respect to the total mass of the neutral acyl lipid, 40% to 100% by mass of the neutral monoacyl lipid was contained, 0% to 60% by mass of the neutral diacyl lipid was contained, and 0% to 20% by mass of the neutral triacyl lipid was contained, the effects were more excellent.

## Claims

1. A biomaterial comprising:
a neutral acyl lipid;
a phospholipid; and
an amphipathic block copolymer which contains a hydrophilic segment and a hydrophobic segment and in which a difference in ClogP value between the hydrophilic segment and the hydrophobic segment is more than 1.00,
wherein a content of the amphipathic block copolymer is 1.0% to 20% by mass with respect to a total mass of a solid content of the biomaterial.

2. The biomaterial according to claim 1,
wherein the amphipathic block copolymer has a number average molecular weight of 1,000 to 10,000, preferably a number average molecular weight of 1,000 to 5,000.

3. The biomaterial according to claim 1 or 2,
wherein the neutral acyl lipid contains 30% to 100% by mass of a neutral monoacyl lipid, 0% to 70% by mass of a neutral diacyl lipid, and 0% to 20% by mass of a neutral triacyl lipid, with respect to a total mass of the neutral acyl lipid.

4. The biomaterial according to any one of claims 1 to 3,
wherein an acyl group of the neutral acyl lipid has 6 to 32 carbon atoms, preferably 16 to 22 carbon atoms.

5. The biomaterial according to any one of claims 1 to 4, further comprising a quaternary ammonium salt.

6. The biomaterial according to claim 5,
wherein a content of the phospholipid and quaternary ammonium salt is 25% to 45% by mass with respect to the total mass of the solid content of the biomaterial.

7. The biomaterial according to any one of claims 1 to 6,
wherein an acyl group of the phospholipid has 12 to 22 carbon atoms, preferably 16 to 18 carbon atoms.

8. The biomaterial according to claim 5,
wherein a quaternary ammonium cation of the quaternary ammonium salt is represented by the following formula, 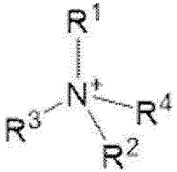 in the formula, R¹, R², R³, and R⁴ are each independently an alkyl group having 1 to 22 carbon atoms, and a hydrogen atom of the alkyl group may be substituted with an alkoxy group, an acyl group, or an acyloxy group.

9. The biomaterial according to claim 8,
wherein the alkoxy group, the acyl group, or the acyloxy group, with which the hydrogen atom of the alkyl group is substituted, has 16 to 18 carbon atoms.

10. The biomaterial according to any one of claims 1 to 9,
wherein the neutral acyl lipid is an acyl glycerol.

11. The biomaterial according to claim 10,
wherein the acyl glycerol is a glyceryl oleate.

12. The biomaterial according to any one of claims 1 to 11,
wherein the difference in ClogP value between the hydrophilic segment and the hydrophobic segment is more than 2.00.

13. The biomaterial according to any one of claims 1 to 12,
wherein the hydrophobic segment contains polycaprolactone.

14. The biomaterial according to any one of claims 1 to 13,
wherein the amphipathic block copolymer is a triblock copolymer.

15. The biomaterial according to claim 14,
wherein the triblock copolymer has a constitution of polycaprolactone-polytetrahydrofuran-polycaprolactone.

16. The biomaterial according to any one of claims 1 to 15,
wherein a content of water is 1.0% by mass or less with respect to a total mass of the biomaterial.

17. The biomaterial according to any one of claims 1 to 16,
wherein the biomaterial forms a liquid crystal phase in a case of being brought into contact with water.

18. The biomaterial according to claim 17,
wherein the liquid crystal phase is any one selected from the group consisting of a hexagonal columnar phase, a lamellar phase, a sponge phase, a bicontinuous cubic phase, and a mixed state of two or more thereof.

19. The biomaterial according to any one of claims 1 to 18,
wherein the biomaterial is used for mucous membrane protection.

20. The biomaterial according to claim 19,
wherein the biomaterial is used for oral cavity mucous membrane protection.

## Patentansprüche

1. Biomaterial, umfassend:
ein neutrales Acyllipid;
ein Phospholipid; und
ein amphipathisches Block-Copolymer, das ein hydrophiles Segment und ein hydrophobes Segment enthält und bei dem ein Unterschied im ClogP-Wert zwischen dem hydrophilen Segment und dem hydrophoben Segment mehr als 1,00 beträgt,
wobei ein Gehalt des amphipathischen Block-Copolymers 1,0 bis 20 Massen-% in Bezug auf eine Gesamtmasse eines Feststoffgehalts des Biomaterials beträgt.

2. Biomaterial nach Anspruch 1,
wobei das amphipathische Block-Copolymer ein Zahlenmittel des Molekulargewichts von 1.000 bis 10.000, vorzugsweise ein Zahlenmittel des Molekulargewichts von 1.000 bis 5.000 aufweist.

3. Biomaterial nach Anspruch 1 oder 2,
wobei das neutrale Acyllipid 30 bis 100 Massen-% eines neutralen Monoacyl-Lipids, 0 bis 70 Massen-% eines neutralen Diacyl-Lipids und 0 bis 20 Massen-% eines neutralen Triacyl-Lipids enthält, in Bezug auf eine Gesamtmasse des neutralen Acyllipids.

4. Biomaterial nach einem der Ansprüche 1 bis 3,
wobei eine Acylgruppe des neutralen Acyllipids 6 bis 32 Kohlenstoffatome, vorzugsweise 16 bis 22 Kohlenstoffatome aufweist.

5. Biomaterial nach einem der Ansprüche 1 bis 4, ferner umfassend ein quartäres Ammoniumsalz.

6. Biomaterial nach Anspruch 5,
wobei ein Gehalt des Phospholipids und des quartären Ammoniumsalzes 25 bis 45 Massen-% in Bezug auf die Gesamtmasse des Feststoffgehalts des Biomaterials beträgt.

7. Biomaterial nach einem der Ansprüche 1 bis 6,
wobei eine Acylgruppe des Phospholipids 12 bis 22 Kohlenstoffatome, vorzugsweise 16 bis 18 Kohlenstoffatome aufweist.

8. Biomaterial nach Anspruch 5,
wobei ein quartäres Ammoniumkation des quartären Ammoniumsalzes durch die folgende Formel dargestellt ist, 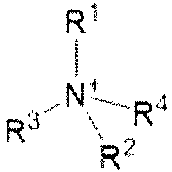 wobei in der Formel R¹, R², R³ und R⁴ jeweils unabhängig voneinander eine Alkylgruppe, die 1 bis 22 Kohlenstoffatome aufweist, sind und ein Wasserstoffatom der Alkylgruppe mit einer Alkoxygruppe, einer Acylgruppe oder einer Acyloxygruppe substituiert sein kann.

9. Biomaterial nach Anspruch 8,
wobei die Alkoxygruppe, die Acylgruppe oder die Acyloxygruppe, mit der das Wasserstoffatom der Alkylgruppe substituiert ist, 16 bis 18 Kohlenstoffatome aufweist.

10. Biomaterial nach einem der Ansprüche 1 bis 9,
wobei das neutrale Acyllipid ein Acylglycerol ist.

11. Biomaterial nach Anspruch 10,
wobei das Acylglycerol ein Glyceryloleat ist.

12. Biomaterial nach einem der Ansprüche 1 bis 11,
wobei der Unterschied im ClogP-Wert zwischen dem hydrophilen Segment und dem hydrophoben Segment mehr als 2,00 beträgt.

13. Biomaterial nach einem der Ansprüche 1 bis 12,
wobei das hydrophobe Segment Polycaprolacton enthält.

14. Biomaterial nach einem der Ansprüche 1 bis 13,
wobei das amphipathische Block-Copolymer ein Triblock-Copolymer ist.

15. Biomaterial nach Anspruch 14,
wobei das Triblock-Copolymer eine Struktur von Polycaprolacton-Polytetrahydrofuran-Polycaprolacton aufweist.

16. Biomaterial nach einem der Ansprüche 1 bis 15,
wobei ein Wassergehalt 1,0 Masse-% oder weniger in Bezug auf eine Gesamtmasse des Biomaterials beträgt.

17. Biomaterial nach einem der Ansprüche 1 bis 16,
wobei das Biomaterial eine Flüssigkristallphase bildet, wenn es mit Wasser in Kontakt gebracht wird.

18. Biomaterial nach Anspruch 17,
wobei die Flüssigkristallphase eine ist, die aus der Gruppe ausgewählt ist, die aus einer hexagonalen kolumnaren Phase, einer lamellaren Phase, einer Schwammphase, einer bikontinuierlichen kubischen Phase und einem gemischten Zustand von zwei oder mehr davon besteht.

19. Biomaterial nach einem der Ansprüche 1 bis 18,
wobei das Biomaterial zum Schleimhautschutz verwendet wird.

20. Biomaterial nach Anspruch 19,
wobei das Biomaterial zum Schutz der Mundhöhlenschleimhaut verwendet wird.

## Revendications

1. Biomatériau comprenant :
un lipide acyle neutre ;
un phospholipide ; et
un copolymère séquencé amphipathique qui contient un segment hydrophile et un segment hydrophobe et dans lequel la différence de valeur ClogP entre le segment hydrophile et le segment hydrophobe est supérieure à 1,00,
la teneur en copolymère séquencé amphipathique étant de 1,0 % à 20 % en masse par rapport à la masse totale de la teneur en matières solides du biomatériau.

2. Biomatériau selon la revendication 1,
ledit copolymère séquencé amphipathique présentant un poids moléculaire moyen en nombre de 1 000 à 10 000, de préférence un poids moléculaire moyen en nombre de 1 000 à 5 000.

3. Biomatériau selon la revendication 1 ou 2,
ledit lipide acyle neutre contenant 30 % à 100 % en masse d'un lipide monoacyle neutre, 0 % à 70 % en masse d'un lipide diacyle neutre et 0 % à 20 % en masse d'un lipide triacyle neutre, par rapport à la masse totale du lipide acyle neutre.

4. Biomatériau selon l'une quelconque des revendications 1 à 3,
un groupe acyle du lipide acyle neutre comportant 6 à 32 atomes de carbone, de préférence 16 à 22 atomes de carbone.

5. Biomatériau selon l'une quelconque des revendications 1 à 4, comprenant en outre un sel d'ammonium quaternaire.

6. Biomatériau selon la revendication 5,
la teneur en phospholipide et en sel d'ammonium quaternaire étant de 25 % à 45 % en masse par rapport à la masse totale de la teneur en matières solides du biomatériau.

7. Biomatériau selon l'une quelconque des revendications 1 à 6,
un groupe acyle du phospholipide comportant 12 à 22 atomes de carbone, de préférence 16 à 18 atomes de carbone.

8. Biomatériau selon la revendication 5,
un cation ammonium quaternaire du sel d'ammonium quaternaire étant représenté par la formule suivante, 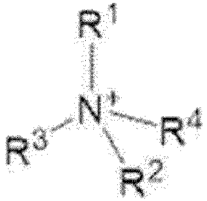 dans la formule, R¹, R², R³ et R⁴ représentent chacun indépendamment un groupe alkyle comportant 1 à 22 atomes de carbone, et un atome d'hydrogène du groupe alkyle peut être substitué par un groupe alcoxy, un groupe acyle ou un groupe acyloxy.

9. Biomatériau selon la revendication 8,
ledit groupe alcoxy, ledit groupe acyle ou ledit groupe acyloxy, par lequel l'atome d'hydrogène du groupe alkyle est substitué, comportant 16 à 18 atomes de carbone.

10. Biomatériau selon l'une quelconque des revendications 1 à 9,
ledit lipide acyle neutre étant un acylglycérol.

11. Biomatériau selon la revendication 10,
ledit acylglycérol étant un oléate de glycéryle.

12. Biomatériau selon l'une quelconque des revendications 1 à 11,
ladite différence de valeur ClogP entre le segment hydrophile et le segment hydrophobe étant supérieure à 2,00.

13. Biomatériau selon l'une quelconque des revendications 1 à 12,
ledit segment hydrophobe contenant de la polycaprolactone.

14. Biomatériau selon l'une quelconque des revendications 1 à 13,
ledit copolymère séquencé amphipathique étant un copolymère triséquencé.

15. Biomatériau selon la revendication 14,
ledit copolymère triséquencé présentant une constitution de polycaprolactone-polytétrahydrofurane-polycaprolactone.

16. Biomatériau selon l'une quelconque des revendications 1 à 15,
la teneur en eau étant de 1,0% en masse ou moins par rapport à la masse totale du biomatériau.

17. Biomatériau selon l'une quelconque des revendications 1 à 16,
ledit biomatériau formant une phase de cristaux liquides en cas de mise en contact avec de l'eau.

18. Biomatériau selon la revendication 17,
ladite phase de cristaux liquides étant l'une quelconque choisie dans le groupe constitué par une phase colonnaire hexagonale, une phase lamellaire, une phase spongieuse, une phase cubique bicontinue et un état mixte d'au moins deux de celles-ci.

19. Biomatériau selon l'une quelconque des revendications 1 à 18,
ledit biomatériau étant utilisé pour la protection des muqueuses.

20. Biomatériau selon la revendication 19,
ledit biomatériau étant utilisé pour la protection de la muqueuse de la cavité buccale.
